(19) Europäisches Patentamt
European Patent Office
Office européen des brevets

(11) Publication number: **0 310 999 B1**

(12) **EUROPEAN PATENT SPECIFICATION**

(45) Date of publication of patent specification: **22.07.92**

(51) Int. Cl.[5]: **A61K 47/02**, A61K 47/06, A61K 31/445

(21) Application number: **88116367.9**

(22) Date of filing: **04.10.88**

(54) **Pharmaceutical composition for piperidinoalkanol derivatives.**

(30) Priority: **07.10.87 US 105928**
**04.11.87 US 117166**
**05.02.88 US 152689**

(43) Date of publication of application:
**12.04.89 Bulletin 89/15**

(45) Publication of the grant of the patent:
**22.07.92 Bulletin 92/30**

(84) Designated Contracting States:
**AT BE CH DE ES FR GB GR IT LI LU NL SE**

(56) References cited:
**EP-A- 0 264 259**
**FR-A- 2 453 854**

(73) Proprietor: **MERRELL DOW PHARMACEUTICALS INC.**
**2110 East Galbraith Road**
**Cincinnati Ohio 45215-6300(US)**

(72) Inventor: **Domet, Jack**
**2158 Flowerwood Court**
**Cincinnati Ohio 45230(US)**
Inventor: **Attarchi, Faraneh**
**35 Clay Court**
**Zionsville Indiana 46077(US)**
Inventor: **Shah, Dhiren N.**
**294 McKenzie Drive**
**West Chester Pennsylvania 19380(US)**

(74) Representative: **Vossius & Partner**
**Siebertstrasse 4 P.O. Box 86 07 67**
**W-8000 München 86(DE)**

**EP 0 310 999 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Various piperidinoalkanol derivatives are disclosed in U.S. Patents 3,878,217, 4,254,129, and 4,285,957 as compounds useful as antihistamines, antiallergy agents, and bronchodilators. Included within the scope of these generically defined piperidinoalkanols is α-[4-(1,1-dimethylethyl)phenyl]-4-(hydroxydiphenylmethyl)-1-piperidinebutanol which is commercially available as a pharmaceutical composition in solid unit dosage form for the treatment of patients with symptoms of seasonal allergic rhinitis.

In general, these piperidinoalkanol derivatives are only minimally soluble in water and therefore the therapeutically inactive ingredients in a pharmaceutical composition containing one or more of these compounds are very important in providing for their efficient and immediate absorption and bioavailability after oral administration.

A novel pharmaceutical composition is now provided which allows efficient and immediate absorption and bioavailability of these compounds after oral administration thereof.

The present invention relates to a pharmaceutical composition for oral administration of various piperidino-alkanol derivatives which are disclosed in U.S. Patents 3,878,217, 4,254,129, and 4,285,957. These piperidino-alkanol derivatives are useful as antihistamines, antiallergy agents, and bronchodilators and are described by the formula (1),

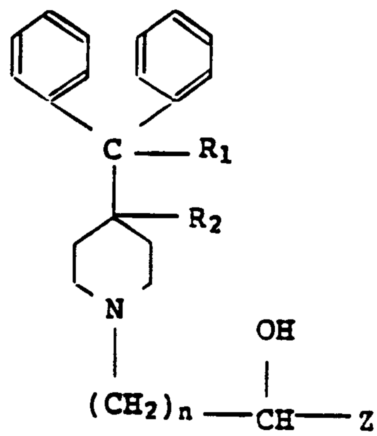

(1)

wherein $R_1$ is hydrogen or hydroxy; $R_2$ is hydrogen; or $R_1$ and $R_2$ taken together form a second bond between the carbon atoms bearing $R_1$ and $R_2$; n is a integer of from 1 to 5; in case n is an integer of from 1 to 3, Z is thienyl, phenyl or substituted phenyl wherein the substituents on the substituted phenyl may be attached at the ortho, meta, or para positions of the substituted phenyl ring and are selected from the group consisting of a halogen atom, a straight or branched lower alkyl chain of from 1 to 4 carbon atoms, a lower alkoxy group of from 1 to 4 carbon atoms, a di(lower alkyl)amino group, or a saturated monocyclic heterocyclic ring selected from pyrrolidino, piperidino, morpholino, or N-(lower alkyl)piperizino; or in case n is an integer of from 1 to 5, Z is

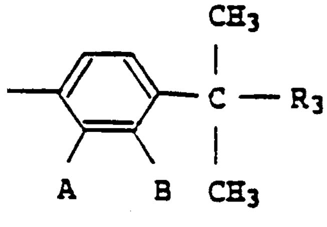

wherein $R_3$ is $-CH_3$, $-CH_2OH$, -COOH or -COOalkyl wherein the alkyl moiety has from 1 to 6 carbon atoms and is straight or branched; each of A and B is hydrogen or hydroxy; with the provisos that at least one of A

or B is hydrogen and one of A or B is other than hydrogen when $R_3$ is $-CH_3$; and pharmaceutically acceptable salts and individual optical isomers thereof.

The pharmaceutical compositions for oral administration provided by the present invention comprise a therapeutically-effective amount of at least one compound of formula (1) associated with calcium carbonate in an amount ranging from about 2% to about 50% by weight of the composition, and a nonionic or a cationic surfactant in an amount ranging from about 0.1% to about 6% by weight of the composition. In addition, the pharmaceutical compositions of the present invention can optionally contain one or more other therapeutically inert ingredients as are well known and appreciated in the art of pharmaceutical science.

The pharmaceutical composition of the present invention is administered orally in the form of tablets, coated tablets, powders, dragees, hard or soft gelatin capsules, solutions, emulsions, or suspensions. The preferred pharmaceutical compositions of the present invention are those in solid unit dosage form such as tablets, coated tablets and capsules. A unit dose is that amount of the pharmaceutical composition which is individually administered.

A therapeutically-effective amount of a compound of formula (1) is that amount which produces the desired therapeutic response (i.e., antihistaminic, anti-allergic, or bronchodilatory effect) upon oral administration according to a single or multiple dosage regimen. An effective amount may vary over a wide range from 0.01 to 20 milligrams (mg) per kilogram (kg) of body weight per dose. A pharmaceutical composition which provides from 10 mg to 150 mg per unit dose is preferred. Pharmaceutical compositions which provide from 40 mg to 70 mg per unit dose and those which provide from 110 mg to 130 mg are especially preferred. The compound α-[4-(1,1-dimethylethyl)phenyl]-4-(hydroxydiphenylmethyl)-1-piperidinebutanol is the preferred therapeutically active ingredient.

According to the present invention, the term "nonionic surfactant" means and includes pharmaceutically acceptable nonionic surfactants known in the art of pharmaceutical science, including various nonionic compounds containing relatively hydrophilic and relatively hydrophobic regions. Typically these surfactants are alkoxylates of hydrophobic amines, acids or alcohols. For example, the term "pharmaceutically acceptable nonionic surfactants" is contemplated to include the following agents within its scope: various long chain fatty acid esters of polyoxyethylene sorbitan, such as Polysorbate 80 (also known as Tween 80); various poloxamers or pluronics, such as Pluronic-F68, polyethylene glycols of various average molecular weights, and derivatives thereof such as polyoxyethylene fatty acid esters (for example polyethylene glycol monostearate); or mixtures thereof. The preferred nonionic surfactants in the pharmaceutical composition of the present invention are polyoxyethylene sorbitan fatty acid esters and polyethylene glycol (average molecular weight 4000 to 9000). Polysorbate 80 and polyethylene glycol (average molecular weight about 8000) are especially preferred.

According to the present invention, the term "cationic surfactant" means and includes various ionic compounds with a positively charged ionic species containing relatively hydrophobic regions. Typically these surfactants are quaternary ammonium salts, such as for example, cetylpyridium chloride, cetyl trimethyl ammonium bromide and benzalkonium chloride. The preferred cationic surfactant for purposes of the present invention is cetylpyridium chloride.

The amount of the nonionic or cationic surfactant in the pharmaceutical composition of the present invention can vary from 0.1% to 6% by weight. The preferred amount is from 0.5% to 4% by weight with from 1% to 3% being most preferred.

The amount of calcium carbonate present in the pharmaceutical composition of the present invention can vary from 2% to 50% by weight. The preferred amount of calcium carbonate is from 2% to 25% by weight with from 12% to 15% being most preferred. It is readily apparent to one skilled in the art that the calcium carbonate may be replaced in whole or in part by other pharmaceutically acceptable carbonate or bicarbonate salts, such as sodium bicarbonate. Calcium carbonate is preferred because of its advantageous handling characteristics (i.e., less sensitive to high humidity).

The pharmaceutical composition of the present invention can optionally contain one or more other therapeutically inert ingredients such as are well known and appreciated in the art. Such therapeutically inert ingredients include: binders such as pregelatinized starch, povidone, cellulose derivatives including methylcellulose or hydroxypropyl methylcellulose; conventional carriers and fillers such as lactose, corn starch or microcrystalline cellulose; lubricants such as magnesium stearate, calcium stearate, zinc stearate, stearic acid, talc or hydrogenated vegetable oil; glidants such as silicon dioxide; disintegrents such as corn starch derivatives (e.g., starch glycolate sodium); sweetening agents; coloring agents; flavoring agents or antioxidants. These additional ingredients can be present in amounts up to about 95% of the total composition weight. Selection of a particular ingredient or ingredients and the amounts used can be readily determined by one skilled in the art by reference to standard procedures and practices with respect to the particular dosage form selected. A preferred combination of additional ingredients for a solid unit dosage

form include pregelatinized corn starch, microcrystalline cellulose, starch glycolate sodium, and magnesium stearate in preferred amounts of about 25 to 45%, 20 to 40%, 1 to 10%, and 0.1 to 1.0%, respectively, with about 30%, 35%, 5%, and 0.55% of each, respectively, being most preferred. Another preferred combination of additional ingredients for a solid unit dosage form includes microcrystalline cellulose, hydroxypropylmethylcellulose having a methoxyl content of from 28% to 30% and a hydroxypropoxyl content of from 7% to 12% and a viscosity of about 5 mPa•s (cps), magnesium stearate, and silicon dioxide amorphous in preferred amounts of about 50% to 70%, 1% to 5%, 0.5 to 2%, and 0.1% to 1%, respectively with about 59%, 3%, 1% and 0.3%, respectively, being most preferred. The above amounts represent percent by weight of the composition.

The ingredients of the pharmaceutical composition according to the present invention are brought together into a dosage form for oral administration according to standard practices and procedures well known in the art of pharmaceutical science using conventional formulation and manufacturing techniques.

In a preferred embodiment of the present invention, solid dosage units are formulated and manufactured in tablet form using the following procedure:

An aqueous solution of the nonionic or cationic surfactant and other water-soluble ingredients (such as HPMC) is intermixed with a mixture of active ingredient (such as α-[4-(1,1-dimethylethyl)phenyl]-4-(hydroxydiphenylmethyl)-1-piperidinebutanol), microcrystalline cellulose, calcium carbonate (total amount or a portion), and pregelatinized corn starch (if any). The granules thus formed are dried and milled to uniform size and then intermixed with the remaining ingredients, such as any remaining portion of calcium carbonate, silicon dioxide amorphous, pregelatinized corn starch, starch glycolate sodium and magnesium stearate. The complete mixture is then subjected to tableting in conventional tableting machines under conventional conditions.

It is of course understood that tablets produced according to the present invention can be film or sugar coated using standard ingredients and procedures commonly used and well known in the art of pharmaceutical science. It is contemplated that tablets so coated are within the scope of the present invention.

The pharmaceutical composition of the present invention demonstrates acceptable *in vitro* dissolution characteristics which indicate that the composition provides efficient bioavailability of the therapeutically active ingredient in an immediate release manner. According to the present invention it is understood that the term "immediate release" contemplates a biopharmaceutical concept indicating the absence of delayed release characteristics.

The following examples are illustrative of preferred embodiments of the present invention and are not intended to limit the scope of the present invention in any way:

## EXAMPLE 1

### 60 mg TABLETS FOR ORAL ADMINISTRATION

Combine 360 grams (g) of α-(4-(1,1-dimethylethyl)phenyl]-4-(hydroxydiphenylmethyl)-1-piperidinebutanol, 1150 g of microcrystalline cellulose, 495 g of calcium carbonate (heavy), and 743 g of pregelatinized corn starch and blend in a mixer for 10 minutes (min). To this mixture add a solution of 99 g of Polysorbate 80 in 1.9 liters of purified water and continue mixing until a good granulation is formed.

Pass the granulation through a 1.68 mm (10-mesh) screen and dry at 49 °C (120 °F) for about 17 hours to a moisture content of about 2.0% to about 2.5% as measured by an O'Haus moisture meter under metering conditions of 40 volts for 20 min. Pass the dried granulation through a 14-mesh screen.

To the granulation add 264 g of pregelatinized corn starch, 165 g of starch glycolate sodium, and 18.15 g of magnesium stearate and mix for about 2 min.

Compress tablets using a tablet press at a weight of 550 milligrams and a hardness of about 88 to 98 N (9 to 10 kp) using 1.0 cm (13/32 inch), round, flat-face, beveled edge tooling.

This procedure results in about 6000 tablets of the following composition:

| INGREDIENT | AMOUNT mg/tablet | COMPOSITION % by weight |
|---|---|---|
| Therapeutically Active Ingredient* | 60.0 | 10.9 |
| Polysorbate 80 | 16.5 | 3.0 |
| Calcium Carbonate | 82.5 | 15.0 |
| Microcrystalline cellulose | 192.5 | 35.0 |
| Pregelatinized Corn Starch | 167.8 | 30.5 |
| Starch Glycolate Sodium | 27.5 | 5.0 |
| Magnesium Stearate | 3.025 | 0.55 |

*α-[4-(1,1-dimethylethyl)phenyl]-4-(hydroxydiphenylmethyl)-1-piperidinebutanol

Use the U.S.P. Paddle Method to determine the dissolution characteristics of the tablets made by the above method. Place a tablet in a U.S.P. Rotating Paddle Apparatus set at 50 revolutions-per-minute (rpm) in 900 milliliters (ml) of de-aerated 0.1 N hydrochloric acid maintained at 37∓5 degrees Celsius (°C). After 60 min withdraw an aliquot of the dissolution medium and assay for α-[4-(1,1-dimethylethyl)phenyl]-4-(hydroxydiphenylmethyl)-1-piperidinebutanol by High Performance Liquid Chromatography (HPLC).

The results of the dissolution averaged for 6 tablets indicate that, after 60 min, 87.8% of the α-[4-(1,1-dimethylethyl)phenyl]-4-(hydroxydiphenylmethyl)-1-piperidinebutanol is released into the dissolution medium (range 86.1% to 89.2%).

Tablets with the same composition as above except that cetylpyridium chloride is used instead of polysorbate 80 are prepared in a manner analogous to that described above. The results of the dissolution averaged for 3 such tablets indicate that, after 60 min, 103% of the α-[4-(1,1-dimethylethyl)phenyl]-4-(hydroxydiphenylmethyl)-1-piperidinebutanol is released into the dissolution medium (range 102% to 105%).

## EXAMPLE 2

### 120mg Tablets for Oral Administration

Combine 4.32 kg of a-[4-(1,1-dimethylethyl)phenyl]-4-(hydroxydiphenylmethyl)-1-piperidinebutanol, 10.57 kg of microcrystalline cellulose and 1.44 kg of calcium carbonate (heavy) and blend in a mixer for about 7 min using a chopper. To this mixture slowly add a solution of 0.54 kg of hydroxypropylmethylcellulose (2910; 5 mPa•s (cps)) and 0.18 kg of polyethylene glycol (8000 powder) in 7.9 liters of purified water while continuing to mix. Continue mixing for about 2 min and slowly add enough purified water to give a good granulation (about 0.5 liter for a 18 kg batch). Continue mixing for an additional 3 min.

Dry the granulation in a fluid bed dryer at 70 degrees centigrade (°C) to a moisture content of about 1.0% to about 2.0% as measured by a computrac moisture analyzer at 115°C. Pass the dried granulation through a 2A.125 screen. Blend the dried granulation for about 5 min with a mixture of 0.72 kg $CaCO_3$ and 0.054 kg Amorphous silicon dioxide, which has been passed through a 0.841 mm (20 mesh) screen. Add 0.18 kg of magnesium stearate that has been passed through a 0.4 mm (40 mesh) screen to the above mix and blend for about 5 min.

Compress tablets at a weight of 500 milligrams and a hardness of about 118 to 147 N (12 to 15 kp) using a suitable tablet press.

This procedure results in about 1000 tablets of the following composition:

| INGREDIENT | AMOUNT mg/tablet | COMPOSITION % by weight |
|---|---|---|
| Therapeutically Active Ingredient[a] | 120 | 24.0 |
| Polyethylene glycol (8000)[b] | 5 | 1.0 |
| Calcium Carbonate | 60 | 12.0 |
| Microcrystalline cellulose | 293.5 | 58.7 |
| HPMC (2910, 5 mPa•s (cps))[c] | 15 | 3.0 |
| Magnesium stearate | 5 | 1.0 |
| Silicon dioxide Amorphous | 1.5 | 0.3 |

[a] α-[4-(1,1-dimethylethyl)phenyl]-4-(hydroxydiphenylmethyl)-1-piperidinebutanol
[b] Polyethylene glycol with an average molecular weight of about 8000
[c] Eydroxypropylmethylcellulose having a methoxyl content of from about 28% to about 30% and a hydroxypropoxyl content of from about 7% to about 12% and a viscosity of about 5 mPa•s (cps).

Use the U.S. P. Paddle Method to determine the dissolution characteristics of the tablets made by the above method. Place a tablet in a U.S.P. Rotating Paddle Apparatus set at 50 revolutions-per-minute (rpm) in 900 milliliters (ml) of de-aerated 0.1 N hydrochloric acid maintained at 37 + 5 ° C. After 45 min withdraw an aliquot of the dissolution medium and assay for α-[4-(1,1-dimethylethyl)phenyl]-4-(hydroxydiphenylmethyl)-1-piperidinebutanol by HPLC.

The results of the dissolution averaged for 6 tablets indicate that, after 45 min, 80.0% of the a-[4-(1,1-dimethylethyl)phenyl]-4-(hydroxydiphenylmethyl)-1-piperidinebutanol is released into the dissolution medium (range 78.3% to 85.9%).

Tablets with the same composition as above except that HPMC (2910, 5 mPa•s (cps)) is 1.6% by weight and microcrystalline cellulose is 60.1% by weight are prepared in a manner analogous to that described above. The results of the dissolution averaged for 6 such tablets indicate that, after 45 min, 81.7% of the a-[4-(1,1-dimethylethyl)phenyl]-4-(hydroxydiphenylmethyl)-1-piperidinebutanol is released into the dissolution medium (range 78.5% to 85.5%).

## Claims

**Claims for the following Contracting States : AT, BE, CH, DE, FR, GB, IT, LI, LU, NL, SE**

1. A pharmaceutical composition in solid unit dosage form comprising a) a therapeutically effective amount of at least one piperidinoalkanol compound of the formula (1),

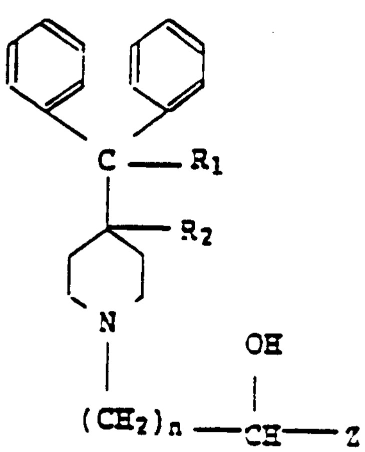

(1)

wherein $R_1$ is hydrogen or hydroxy; $R_2$ is hydrogen; or $R_1$ and $R_2$ taken together form a second bond between the carbon atoms bearing $R_1$ and $R_2$; n is an integer of from 1 to 5; in case n is an integer of

from 1 to 3, Z is thienyl, phenyl or substituted phenyl wherein the substituents on the substituted phenyl may be attached at the ortho, meta, or para positions of the substituted phenyl ring and are selected from a halogen atom, a straight or branched lower alkyl chain of from 1 to 4 carbon atoms, a lower alkoxy group of from 1 to 4 carbon atoms, a di(lower alkyl)amino group, or a saturated monocyclic heterocyclic ring selected from pyrrolidino, piperidino, morpholino, or N-(lower alkyl)-piperizino; or in case n is an integer of from 1 to 5, Z is

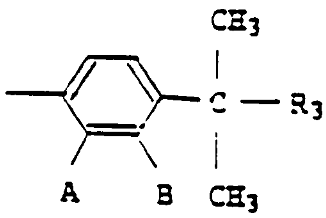

wherein $R_3$ is $-CH_3$, $-CH_2OH$, -COOH or -COOalkyl wherein the alkyl moiety has from 1 to 6 carbon atoms and is straight or branched; each of A and B is hydrogen or hydroxy; with the provisos that at least one of A or B is hydrogen and one of A or B is other than hydrogen when $R_3$ is $-CH_3$; and pharmaceutically acceptable salts and individual optical isomers thereof, b) one or more nonionic surfactants in an amount of from 0.1% to 6% by weight of the composition, c) calcium carbonate in an amount of from 2% to 50% by weight of the composition.

**2.** A pharmaceutical composition according to Claim 1 wherein the piperidinoalkanol is α-[4-(1,1-dimethylethyl)phenyl]-4-(hydroxydiphenylmethyl)-1-piperidinebutanol.

**3.** A composition according to Claim 2 wherein the α-[4-(1,1-dimethylethyl)phenyl]-4-(hydroxydiphenylmethyl)-1-piperidinebutanol is present in the amount of about 60 mg.

**4.** A composition according to Claim 2 wherein the α-[4-(1,1-dimethylethyl)phenyl]-4-(hydroxydiphenylmethyl)-1-piperidinebutanol is present in the amount of about 120 mg.

**5.** A composition according to Claim 3 or 4 wherein the nonionic surfactant is present in an amount of from 0.5% to 4% by weight of the composition.

**6.** A composition according to Claim 3 or 4 wherein the nonionic surfactant is present in an amount of from 1% to 3% by weight of the composition.

**7.** A composition according to Claim 3 wherein the nonionic surfactant is Polysorbate 00.

**8.** A composition according to Claim 4 wherein the nonionic surfactant is polyethylene glycol.

**9.** A composition according to Claim 3 or 4 wherein calcium carbonate is present in an amount of from 2% to 25% by weight of the composition.

**10.** A composition according to Claim 3 or 4 wherein the calcium carbonate is present in an amount of from 12% to 15% by weight of the composition.

**11.** A composition according to Claim 3 which comprises Polysorbate 80 and calcium carbonate in amounts of about 3% and 15%, respectively, by weight of the composition and further comprises, microcrystalline cellulose, pregelatinized corn starch, sodium starch glycolate and magnesium stearate in amounts of about 35%, 30.5%, 5%, and 0.55%, respectively, by weight of the composition.

**12.** A composition according to Claim 4 which comprises polyethylene glycol and calcium carbonate in amounts of about 1% and 12%, respectively, by weight of the composition and further comprises microcrystalline cellulose, HPMC, magnesium stearate, and silicon dioxide in amounts of about 60.4%, 1.6%, 1% and 0.3%, respectively, by weight of the composition.

**13.** A composition according to Claim 4 which comprises polyethylene glycol and calcium carbonate in amounts of about 1% and 12%, respectively, by weight off the composition and further comprises microcrystallin cellulose, HPMC, magnesium stearate, and silicon dioxide in amounts of about 59%, 3%, 1% and 0.3%, respectively, by weight of the composition.

**14.** A pharmaceutical composition in solid unit dosage form comprising a) therapeutically effective amounts of at least one piperidinoalkanol compound as shown in Claim 1, b) calcium carbonate in amount of from 2% to 50% by weight of the composition, and c) one or more cationic surfactants in an amount of from 0.1% to 6% by weight of the composition.

**15.** A composition according to Claim 14 wherein the piperidinoalkanol compound is α-[4-(1,1-dimethylethyl)phenyl)-4-(hydroxydiphenylmethyl)-1-piperidinebutanol.

**16.** A composition according to Claim 14 or 15 wherein the cationic surfactant is cetylpyridium chloride.

**17.** A composition according to Claim 14 or 15 wherein the cationic surfactant comprises about 3% by weight of the composition.

**18.** A composition according to Claim 16 which comprises cetylpyridium chloride and calcium carbonate in amounts of about 3%, and 15%, respectively, by weight of the composition and further comprises micro-crystalline cellulose, pregelatinized corn starch, sodium starch glycolate and magnesium stearate in amounts of about 35%, 30.5%, 5%, and 0.55%, respectively, by weight of the composition.

**19.** A process for making a pharmaceutical composition comprising combining a) a therapeutically effective amount of at least one piperidinoalkanol compound as shown in Claim 1, b) one or more nonionic surfactants in an amount of from 0.1% to 6% by weight of the composition, c) calcium carbonate in an amount of from 2% to 50% by weight of the composition.

**20.** A process according to Claim 19 which further comprises compressing the pharmaceutical composition into a solid unit dosage form.

**Claims for the following Contracting States : ES, GR**

**1.** A process for making a pharmaceutical composition comprising combining a) a therapeutically effective amount of at least one piperidinoalkanol compound of the formula (1),

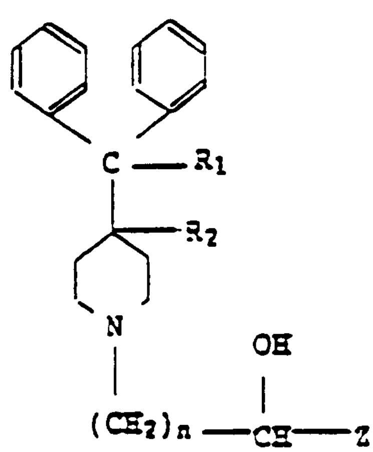

(1)

wherein $R_1$ is hydrogen or hydroxy; $R_2$ is hydrogen; or $R_1$ and $R_2$ taken together form a second bond between the carbon atoms bearing $R_1$ and $R_2$; n is an integer of from 1 to 5; in case n is an integer of from 1 to 3, Z is thienyl, phenyl or substituted phenyl wherein the substituents on the substituted phenyl may be attached at the ortho, meta, or para positions of the substituted phenyl ring and are selected from a halogen atom, a straight or branched lower alkyl chain of from 1 to 4 carbon atoms, a

lower alkoxy group of from 1 to 4 carbon atoms, a di(lower alkyl)amino group, or a saturated monocyclic heterocyclic ring selected from pyrrolidino, piperidino, morpholino, or N-(lower alkyl)-piperizino; or in case n is an integer of from 1 to 5, Z is

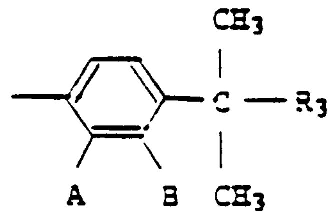

wherein $R_3$ is $-CH_3$, $-CH_2OH$, -COOH or -COOalkyl wherein the alkyl moiety has from 1 to 6 carbon atoms and is straight or branched; each of A and B is hydrogen or hydroxy; with the provisos that at least one of A or B is hydrogen and one of A or B is other than hydrogen when $R_3$ is $-CH_3$; and pharmaceutically acceptable salts and individual optical isomers thereof, b) one or more nonionic surfactants in an amount of from 0.1% to 6% by weight of the composition, c) calcium carbonate in an amount of from 2% to 50% by weight of the composition.

**2.** A process according to Claim 1 which further comprises compressing the pharmaceutical composition into a solid unit dosage form.

**3.** A process according to Claim 1 or 2 wherein the piperidinoalkanol is α-[4-(1,1-dimethylethyl)phenyl]-4-(hydroxydiphenylmethyl)-1-piperidinebutanol.

**4.** A process according to Claim 3 wherein the α-[4-(1,1-dimethylethyl)phenyl]-4-(hydroxydiphenylmethyl)-1-piperidinebutanol is used in the amount of about 60 mg.

**5.** A process according to Claim 3 wherein the α-[4-(1,1-dimethylethyl)phenyl]-4-(hydroxydiphenylmethyl)-1-piperidinebutanol is used in the amount of about 120 mg.

**6.** A process according, to Claim 4 or 5 wherein the nonionic surfactant is used in an amount of from 0.5% to 4% by weight of the composition.

**7.** A process according to Claim 4 or 5 wherein the nonionic surfactant is used in an amount of from 1% to 3% by weight of the composition.

**8.** A process according to Claim 4 wherein the nonionic surfactant is Polysorbate 80.

**9.** A process according to Claim 5 wherein the nonionic surfactant is polyethylene glycol.

**10.** A process according to Claim 4 or 5 wherein calcium carbonate is used in an amount of from 2% to 25% by weight of the composition.

**11.** A process according to Claim 4 or 5 wherein the calcium carbonate is used in an amount of from 12% to 15% by weight of the composition.

**12.** A process according to Claim 4 which comprises combining Polysorbate 80 and calcium carbonate in amounts of about 3% and 15%, respectively, by weight of the composition and further comprises, microcrystalline cellulose, pregelatinized corn starch, sodium starch glycolate and magnesium stearate in amounts of about 35%, 30.5%, 5%, and 0.55%, respectively, by weight of the composition.

**13.** A process according to Claim 5 which comprises combining polyethylene glycol and calcium carbonate in amounts of about 1% and 12%, respectively, by weight of the composition and further comprises microcrystalline cellulose, HPMC, magnesium stearate, and silicon dioxide in amounts of about 60.4%, 1.6%, 1% and 0.3%, respectively, by weight of the composition.

**14.** A process according to Claim 5 which comprises combining polyethylene glycol and calcium carbonate in amounts of about 1% and 12% respectively, by weight of the composition and further comprises microcrystalline cellulose, HPMC, magnesium stearate, and silicon dioxide in amounts of about 59%, 3%, 1% and 0.3%, respectively, by weight of the composition.

**15.** A process for making a pharmaceutical composition in solid unit dosage form comprising combining a) therapeutically effective amounts of at least one piperidinoalkanol compound as shown in claim 1, b) calcium carbonate in an amount of from 2% to 50% by weight of the composition, and c) one or more cationic surfactants in an amount of from 0.1% to 6% by weight of the composition.

**16.** A process according to claim 15 which further comprises compressing the pharmaceutical composition into a solid unit dosage form.

**17.** A process according to Claim 15 or 16 wherein the piperidinoalkanol compound is $\alpha$-[4-(1,1-dimethylethyl)phenyl]-4-(hydrorydiphenyimethyl)-1-piperidinebutanol.

**18.** A process according to Claim 16 or 17 wherein the cationic surfactant is cetylpyridium chloride.

**19.** A process according to Claim 16 or 17 wherein the cationic surfactant comprises about 3% by weight of the composition.

**20.** A process according to claim 18 which comprises combining cetylpyridium chloride and calcium carbonate in amounts of about 3%, and 15%, respectively, by weight of the composition and further comprises micro-crystalline cellulose, pregelatinized corn starch, sodium starch glycolate and magnesium stearate in amounts of about 35%, 30.5%, 5%, and 0.55%, respectively, by weight of the composition.

**Revendications**

**Revendications pour les Etats contractants suivants : AT, BE, CH, DE, FR, GB, IT, LI, LU, NL, SE**

**1.** Une composition pharmaceutique sous forme de dosage unitaire solide comprenant a) une quantité thérapeutiquement efficace d'au moins un composé pipéridinoalcanol de formule (1),

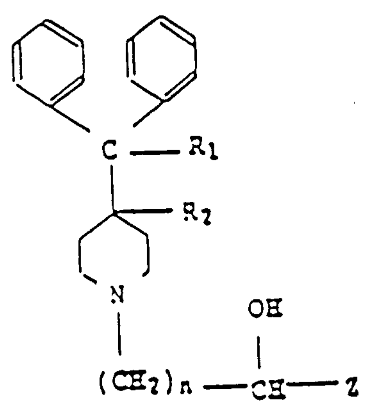

(1)

dans laquelle $R_1$ est un atome d'hydrogène ou un groupe hydroxy ; $R_2$ est un atome d'hydrogène ; ou bien $R_1$ et $R_2$ pris ensemble forment une seconde liaison entre les atomes de carbone portant $R_1$ et $R_2$ ; n est un nombre entier de 1 à 5 ; dans le cas où n est un nombre entier de 1 à 3, Z est un groupe thiényle, phényle ou phényle substitué dont les substituants sur le groupe phényle substitué peuvent être liés aux positions ortho, méta ou para du noyau phényle substitué et sont choisis parmi un atome d'halogène, un groupe alkyle inférieur à chaîne droite ou ramifiée en $C_1$-$C_4$, un groupe alcoxy inférieur en $C_1$-$C_4$, un groupe di(alkyl inférieur)amino, ou un noyau hétérocyclique monocyclique saturé choisi parmi les suivants : pyrrolidino, pipéridino, morpholino ou N-(alkyl inférieur)pipérazino ; ou dans le cas où n est un nombre entier de 1 à 5, Z est

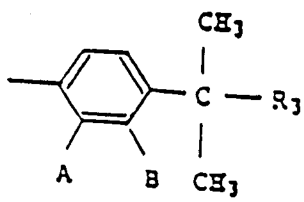

dans laquelle $R_3$ est $-CH_3$, $-CH_2OH$, -COOH ou -COOalkyle où le reste alkyle a de 1 à 6 atomes de carbone et est linéaire ou ramifié ; chacun des A et B est un atome d'hydrogène ou un groupe hydroxy ; à la condition qu'au moins l'un des A ou B est un atome d'hydrogène et l'un des A ou B est autre que l'atome d'hydrogène lorsque $R_3$ est $-CH_3$ ; et les sels pharmaceutiquement acceptables et les isomères optiques individuels dudit composé, b) un ou plusieurs agents surfactants non ioniques en quantité de 0,1 % à 6 % en poids de la composition, c) du carbonate de calcium en quantité de 2 % à 50 % en poids de la composition.

2. Une composition pharmaceutique selon la revendication 1, selon laquelle le pipéridinoalcanol est l'α-[4-(1,1-diméthyléthyl)phényl]-4-(hydroxydiphénylméthyl)-1-pipéridinobutanol.

3. Une composition selon la revendication 2, selon laquelle l'α-[4-(1,1-diméthyléthyl)phényl]-4-(hydroxydiphénylméthyl)-1-pipéridinobutanol est présent en quantité d'environ 60 mg.

4. Une composition selon la revendication 2, selon laquelle l'α-[4-(1,1-diméthyléthyl)phényl]-4-(hydroxydiphénylméthyl)-1-pipéridinobutanol est présent en quantité d'environ 120 mg.

5. Une composition selon la revendication 3 ou 4, selon laquelle le surfactant non ionique est présent en quantité de 0,5 % à 4 % en poids de la composition.

6. Une composition selon la revendication 3 ou 4, selon laquelle le surfactant non ionique est présent en quantité de 1 % à 3 % en poids de la composition.

7. Une composition selon la revendication 3, selon laquelle le surfactant non ionique est le Polysorbate 80.

8. Une composition selon la revendication 4, selon laquelle le surfactant non ionique est le polyéthylèneglycol.

9. Une composition selon la revendication 3 ou 4, selon laquelle le carbonate de calcium est présent en quantité de 2 % à 25 % en poids de la composition.

10. Une composition selon la revendication 3 ou 4, selon laquelle le carbonate de calcium est présent en quantité de 12 % à 15 % en poids de la composition.

11. Une composition selon la revendication 3, qui comprend le Polysorbate 80 et le carbonate de calcium en quantités d'environ 3 % et 15 %, respectivement, en poids de la composition et qui comprend en outre la cellulose microcristalline, l'amidon de maïs prégélatinisé, le glycolate d'amidon sodique et le stéarate de magnésium en quantités d'environ 35 %, 30,5 %, 5 %, et 0,55 %, respectivement, en poids de la composition.

12. Une composition selon la revendication 4, qui comprend le polyéthylèneglycol et le carbonate de calcium en quantités d'environ 1 % et 12 %, respectivement, en poids de la composition et qui comprend en outre la cellulose microcristalline, la HPMC, le stéarate de magnésium et le dioxyde de silicium en quantités d'environ 60,4 %, 1,6 %, 1 % et 0,3 %, respectivement, en poids de la composition.

13. Une composition selon la revendication 4, qui comprend le polyéthylèneglycol et le carbonate de calcium en quantités d'environ 1 % et 12 %, respectivement, en poids de la composition et qui comprend en outre la cellulose microcristalline, la HPMC, le stéarate de magnésium et le dioxyde de

silicium en quantités d'environ 59 %, 3 %, 1 % et 0,3 %, respectivement, en poids de la composition.

**14.** Une composition pharmaceutique sous forme de dosage unitaire solide comprenant a) des quantités thérapeutiquement efficaces d'au moins un composé pipéridinoalcanol comme montré dans la revendication 1, b) du carbonate de calcium en quantité de 2 % à 50 % en poids de la composition et c) un ou plusieurs surfactants cationiques en quantité de 0,1 % à 6 % en poids de la composition.

**15.** Une composition selon la revendication 14, selon laquelle le composé pipéridinoalcanol est l'α-[4-(1,1-diméthyléthyl)phényl]-4-(hydroxydiphénylméthyl)-1-pipéridinobutanol.

**16.** Une composition selon la revendication 14 ou 15, selon laquelle le surfactant cationique est le chlorure de cétylpyridinium.

**17.** Une composition selon la revendication 14 ou 15, selon laquelle le surfactant cationique comprend environ 3 % en poids de la composition.

**18.** Une composition selon la revendication 16, qui comprend du chlorure de cétylpyridinium. et du carbonate de calcium en quantités d'environ 3 % et 15 %, respectivement, en poids de la composition et qui comprend en outre la cellulose microcristalline, l'amidon de maïs prégélatinisé, le glycolate d'amidon sodique, et le stéarate de magnésium en quantités d'environ 35 %, 30,5 %, 5 % et 0,55 %, respectivement, en poids de la composition.

**19.** Un procédé pour fabriquer une composition pharmaceutique consistant à combiner a) une quantité thérapeutiquement efficace d'au moins un composé pipéridinoalcanol comme montré dans la revendication 1, b) un ou plusieurs surfactants non ioniques en quantité de 0,1 % à 6 % en poids de la composition, c) du carbonate de calcium en quantité de 2 % à 50 % en poids de la composition.

**20.** Un procédé selon la revendication 19, qui comprend en outre la compression de la composition pharmaceutique en une forme de dosage unitaire solide.

**Revendications pour les Etats contractants suivants : ES, GR**

**1.** Un procédé de fabrication d'une composition pharmaceutique consistant à combiner a) une quantité thérapeutiquement efficace d'au moins un composé pipéridinoalcanol de formule (1),

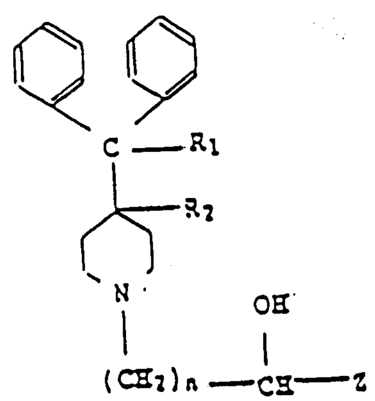

(1)

dans laquelle $R_1$ est un atome d'hydrogène ou un groupe hydroxy ; $R_2$ est un atome d'hydrogène ; ou bien $R_1$ et $R_2$ pris ensemble forment une seconde liaison entre les atomes de carbone portant $R_1$ et $R_2$ ; n est un nombre entier de 1 à 5 ; dans le cas où n est un nombre entier de 1 à 3, Z est un groupe thiényle, phényle ou phényle substitué dont les substituants du groupe phényle substitué peuvent être liés aux positions ortho, méta ou para du noyau phényle substitué et sont choisis parmi un atome d'halogène, un groupe alkyle inférieur à chaîne droite ou ramifiée en $C_1$-$C_4$, un groupe alcoxy inférieur en $C_1$-$C_4$, un groupe di(alkyl inférieur) amino, ou un noyau hétérocyclique monocyclique saturé choisi

parmi les suivants : pyrrolidino, pipéridino, morpholino ou N-(alkyl inférieur)pipérazino ; ou dans le cas où n est un nombre entier de 1 à 5, Z est

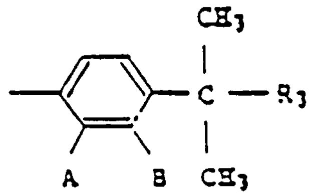

dans laquelle $R_3$ est $-CH_3$, $-CH_2OH$, -COOH ou -COOalkyle où le reste alkyle a de 1 à 6 atomes de carbone et est linéaire ou ramifié ; chacun des A et B est un atome d'hydrogène ou un groupe hydroxy ; à la condition qu'au moins l'un des A ou B est un atome d'hydrogène et l'un des A ou B est autre que l'atome d'hydrogène lorsque $R_3$ est $-CH_3$ ; et les sels pharmaceutiquement acceptables et les isomères optiques individuels dudit composé, b) un ou plusieurs surfactants non ioniques en quantité d'environ 0,1 % à 6 % en poids de la composition, c) du carbonate de calcium en quantité de 2 % à 50 % en poids de la composition.

2. Un procédé selon la revendication 1, qui comprend la compression de la composition pharmaceutique en une forme de dosage unitaire solide.

3. Un procédé selon la revendication 1 ou 2, selon lequel le pipéridinoalcanol est l'α-[4-(1,1-diméthyléthyl)phényl]-4-hydroxydiphénylméthyl)-1-pipéridinobutanol.

4. Un procédé selon la revendication 3, selon lequel l'α-[4-(1,1-diméthyléthyl)phényl]-4-(hydroxydiphénylméthyl)-1-pipéridinobutanol est utilisé en quantité d'environ 60 mg.

5. Un procédé selon la revendication 3, selon lequel l'α-[4-(1,1-diméthyléthyl)phényl)-4-(hydroxydiphénylméthyl)-1-pipéridinobutanol est utilisé en quantité d'environ 120 mg.

6. Un procédé selon la revendication 4 ou 5, selon lequel le surfactant non ionique est utilisé en quantité de 0,5 % à 4 % en poids de la composition.

7. Un procédé selon la revendication 4 ou 5, selon lequel le surfactant non ionique est utilisé en quantité de 1 % à 3 % en poids de la composition.

8. Un procédé selon la revendication 4, selon lequel le surfactant non ionique est le Polysorbate 80.

9. Un procédé selon la revendication 5, selon lequel le surfactant non ionique est le polyéthylèneglycol.

10. Un procédé selon la revendication 4 ou 5, selon lequel le carbonate de calcium est utilisé en quantité de 2 % à 25 % en poids de la composition.

11. Un procédé selon la revendication 4 ou 5, selon lequel le carbonate de calcium est utilisé en quantité de 12 % à 15 % en poids de la composition.

12. Un procédé selon la revendication 4, qui comprend la combinaison du Polysorbate 80 et du carbonate de calcium en quantités d'environ 3 % et 15 %, respectivement, en poids de la composition et qui comprend en outre la cellulose microcristalline, l'amidon de mais prégélatinisé, le glycolate d'amidon sodique et le stéarate de magnésium en quantités d'environ 35 %, 30,5 %, 5 % et 0,55 %, respectivement, en poids de la composition.

13. Un procédé selon la revendication 5, qui consiste à combiner le polyéthylèneglycol et le carbonate de calcium en quantités d'environ 1 % et 12 %, respectivement, en poids de la composition et qui comprend en outre la cellulose microcristalline, la HPMC, le stéarate de magnésium et le dioxyde de silicium en quantités d'environ 60,4 %, 1,6 %, 1 % et 0,3 %, respectivement, en poids de la composition.

**14.** Un procédé selon la revendication 5, qui consiste à combiner le polyéthylèneglycol et le carbonate de calcium en quantités d'environ 1 % et 12 %, respectivement, en poids de la composition et qui comprend en outre la cellulose microcristalline, la HPMC, le stéarate de magnésium et le dioxyde de silicium en quantités d'environ 59 %, 3 %, 1 % et 0,3 %, respectivement, en poids de la composition.

**15.** Un procédé pour fabriquer une composition pharmaceutique sous forme de dosage unitaire solide consistant à combiner a) des quantités thérapeutiquement efficaces d'au moins un composé pipéridinoalcanol comme montré dans la revendication 1, du carbonate de calcium en quantité de 2 % à 50 % en poids de la composition, et c) un ou plusieurs surfactants cationiques en quantité de 0,1 % à 6 % en poids de la composition.

**16.** Un procédé selon la revendication 15, qui comprend en outre la compression de la composition pharmaceutique en forme de dosage unitaire solide.

**17.** Un procédé selon la revendication 15 ou 16, selon lequel le composé pipéridinoalcanol est l'α-[4-(1,1-diméthyléthyl)-phényl]-4-(hydroxydiphénylméthyl)-1-pipéridinobutanol.

**18.** Un procédé selon la revendication 16 ou 17, selon lequel le surfactant cationique est le chlorure de cétylpyridinium.

**19.** Un procédé selon la revendication 16 ou 17, selon lequel le surfactant cationique comprend environ 3 % en poids de la composition.

**20.** Un procédé selon la revendication 18, qui consiste à combiner le chlorure de cétylpyridinium. et le carbonate de calcium en quantités d'environ 3 % et 15 %, respectivement, en poids de la composition et qui comprend en outre la cellulose microcristalline, l'amidon de mais prégélatinisé, le glycolate d'amidon sodique et le stéarate de magnésium en quantités d'environ 35 %, 30,5 %, 5 % et 0,55 %, respectivement, en poids de la composition.

**Patentansprüche**
**Patentansprüche für folgende Vertragsstaaten : AT, BE, CH, DE, FR, GB, IT, LI, LU, NL, SE**

**1.** Arzneimittel in fester Form der Dosierungseinheit, umfassend a) eine therapeutisch wirksame Menge von mindestens einer Piperidinalkanolverbindung der Formel (1)

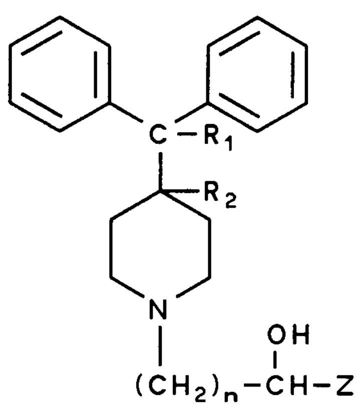

(1)

in der $R_1$ ein Wasserstoffatom oder eine Hydroxylgruppe bedeutet; $R_2$ ein Wasserstoffatom ist; oder $R_1$ und $R_2$ zusammengenommen eine zweite Bindung zwischen den Kohlenstoffatomen, die $R_1$ und $R_2$ tragen, bilden; n eine ganze Zahl von 1 bis 5 ist; falls n eine ganze Zahl von 1 bis 3 ist, Z eine Thienyl-, Phenyl- oder substituierte Phenylgruppe bedeutet, wobei die Substituenten an der substituierten Phenylgruppe an den ortho-` meta oder para-Stellungen des substituierten Phenylringes gebunden sein können und aus einem Halogenatom, einer unverzweigten oder verzweigten Niederalkylkette aus 1 bis 4 Kohlenstoffatomen, einem Niederalkoxyrest aus 1 bis 4 Kohlenstoffatomen, einem Di(niederolkyl)-aminorest oder aus einem gesättigten, monocyclischen, heterocyclischen Ring, ausgewählt aus Pyrrolidin, Piperidin, Morpholin oder N-(Niederalkyl)piperizin, ausgewählt sind, oder falls n eine ganze Zahl von 1 bis 5 ist, Z einen Rest der Formel

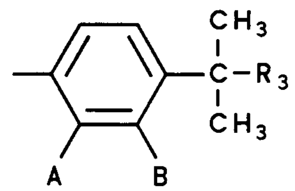

bedeutet, in der $R_3$ einen Rest der Formel $-CH_3$, $-CH_2OH$, -COOH oder der Formel -COOAlkyl bedeutet, in der die Alkyleinheit 1 bis 6 Kohlenstoffatome besitzt und unverzweigt oder verzweigt ist; A und B jeweils ein Wasserstoffatom oder eine Hydroxylgruppe bedeuten; mit den Maßgaben, daß mindestens einer der Reste A oder B ein Wasserstoffatom ist und einer der Reste A oder B kein Wasserstoffatom bedeutet, falls $R_3$ ein Rest der Formel $-CH_3$ ist; und pharmazeutisch verträgliche Salze und einzelne optische Isomere davon, b) ein oder mehrere nichtionische Netzmittel in einer Menge von 0.1 bis 6 Gewichtsprozent der Zusammensetzung, c) Calciumcarbonat in einer Menge von 2 bis 50 Gewichtsprozent der Zusammensetzung.

**2.** Arzneimittel gemäß Anspruch 1, wobei das Piperidinalkanol α-[4-(1,1-Dimethylethyl)phenyl]-4-(hydroxydiphenylmethyl)-1-piperidinbutanol ist.

**3.** Zusammensetzung gemäß Anspruch 2, wobei das α-[4-(1,1-Dimethylethyl)phenyl]-4-(hydroxydiphenylmethyl)-1-piperidinbutanol in der Menge von etwa 60 mg vorliegt.

**4.** Zusammensetzung gemäß Anspruch 2, wobei das α-[4-(1,1-Dimethylethyl)phenyl]-4-(hydroxydiphenylmethyl)-1-piperidinbutanol in der Menge von etwa 120 mg vorliegt.

**5.** Zusammensetzung gemäß Anspruch 3 oder 4, wobei das nichtionische Netzmittel in einer Menge von 0.5 bis 4 Gewichtsprozent der Zusammensetzung vorliegt.

**6.** Zusammensetzung gemäß Anspruch 3 oder 4, wobei das nichtionische Netzmittel in einer Menge von 1 bis 3 Gewichtsprozent der Zusammensetzung vorliegt.

**7.** Zusammensetzung gemäß Anspruch 3, wobei das nichtionische Netzmittel Polysorbat 80 ist.

**8.** Zusammensetzung gemäß Anspruch 4, wobei das nichtionische Netzmittel Polyethylenglykol ist.

**9.** Zusammensetzung gemäß Anspruch 3 oder 4, wobei Calciumcarbonat in einer Menge von 2 bis 25 Gewichtsprozent der Zusammensetzung vorliegt.

**10.** Zusammensetzung gemäß Anspruch 3 oder 4, wobei das Calciumcarbonat in einer Menge von 12 bis 15 Gewichtsprozent der Zusammensetzung vorliegt.

**11.** Zusammensetzung gemäß Anspruch 3, umfassend Polysorbat 80 und Calciumcarbonat in Mengen von etwa 3 beziehungsweise 15 Gewichtsprozent der Zusammensetzung und umfassend ferner mikrokristalline Cellulose, vorgelatinierte Maisstärke, Natriumstärkeglykolat und Magnesiumstearat in Mengen von etwa 35, 30.5, 5 beziehungsweise 0.55 Gewichtsprozent der Zusammensetzung.

**12.** Zusammensetzung gemäß Anspruch 4, umfassend Polyethylenglykol und Calciumcarbonat in Mengen von etwa 1 beziehungsweise 12 Gewichtsprozent der Zusammensetzung und umfassend ferner mikrokristalline Cellulose, HPMC, Magnesiumstearat und Siliciumdioxid in Mengen von etwa 60.4, 1.6, 1 beziehungsweise 0.3 Gewichtsprozent der Zusammensetzung.

**13.** Zusammensetzung gemäß Anspruch 4, umfassend Polyethylenglykol und Calciumcarbonat in Mengen von etwa 1 beziehungsweise 12 Gewichtsprozent der Zusammensetzung und umfassend ferner

mikrokristalline Cellulose, HPMC, Magnesiumstearat und Siliciumdioxid in Mengen von etwa 59, 3, 1 beziehungsweise 0.3 Gewichtsprozent der Zusammensetzung.

**14.** Arzneimittel in fester Form der Dosierungseinheit, umfassend a) therapeutisch wirksame Mengen von mindestens einer Piperidinalkanolverbindung, wie in Anspruch 1 gezeigt, Calciumcarbonat in einer Menge von 2 bis 50 Gewichtsprozent der Zusammensetzung, und c) ein oder mehrere kationische Netzmittel in einer Menge von 0.1 bis 6 Gewichtsprozent der Zusammensetzung.

**15.** Arzneimittel gemäß Anspruch 14, wobei die Piperidinalkanolverbindung α-[4-(1,1-Dimethylethyl)phenyl]-4-(hydroxydiphenylmethyl)-1-piperidinbutanol ist.

**16.** Zusammensetzung gemäß Anspruch 14 oder 15, wobei das kationische Netzmittel Cetylpyridiumchlorid ist.

**17.** Zusammensetzung gemäß Anspruch 14 oder 15, wobei das kationische Netzmittel etwa 3 Gewichtsprozent der Zusammensetzung umfaßt.

**18.** Zusammensetzung gemäß Anspruch 16, umfassend Cetylpyridiumchlorid und Calciumcarbonat in Mengen von etwa 3 beziehungsweise 15 Gewichtsprozent der Zusammensetzung und umfassend ferner mikrokristalline Cellulose, vorgelatinierte Maisstärke, Natriumstärkeglykolat und Magnesiumstearat in Mengen von etwa 35, 30.5, 5 beziehungsweise 0.55 Gewichtsprozent der Zusammensetzung.

**19.** Verfahren zur Herstellung eines Arzneimittels, umfassend die Vereinigung a) einer therapeutisch wirksamen Menge von mindestens einer Piperidinalkanolverbindung, wie in Anspruch 1 gezeigt, b) eines oder mehrerer nichtionischer Netzmittel in einer Menge von 0.1 bis 6 Gewichtsprozent der Zusammensetzung, c) von Calciumcarbonat in einer Menge von 2 bis 50 Gewichtsprozent der Zusammensetzung.

**20.** Verfahren gemäß Anspruch 19, umfassend ferner das Pressen des Arzneimittels in eine feste Form der Dosierungseinheit.

**Patentansprüche für folgende Verttragsstaaten : ES, GR**

**1.** Verfahren zur Herstellung eines Arzneimittels, umfassend die Vereinigung a) einer therapeutisch wirksamen Menge von mindestens einer Piperidinalkanolverbindung der Formel (1)

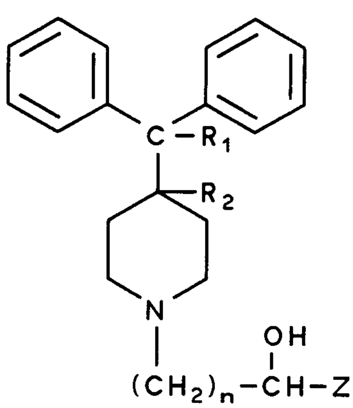

(1)

in der $R_1$ ein Wasserstoffatom oder eine Hydroxylgruppe bedeutet; $R_2$ ein Wasserstoffatom ist; oder $R_1$ und $R_2$ zusammengenommer eine zweite Bindung zwischen den Kohlenstoffatomen, die $R_1$ und $R_2$ tragen, bilden; n eine ganze Zahl von 1 bis 5 ist; falls n eine ganze Zahl von 1 bis 3 ist, Z eine Thienyl-, Phenyl- oder substituierte Phenylgruppe bedeutet, wobei die Substituenten an der substituierten Phenylgruppe an den ortho-, meta oder para-Stellungen des substituierten Phenylringes gebunden sein können und aus einem Halogenatom, einer unverzweigten oder verzweigten Niederalkylkette aus 1 bis 4 Kohlenstoffatomen, einem Niederalkoxyrest aus 1 bis 4 Kohlenstoffatomen, einem Di(niederalkyl)-aminorest oder aus einem gesättigten, monocyclischen, heterocyclischen Ring, ausgewählt aus Pyrrolidin, Piperidin, Morpholin oder N-(Niederalkyl)piperizin, ausgewählt sind, oder falls n eine ganze Zahl von 1 bis 5 ist, Z einen Rest der Formel

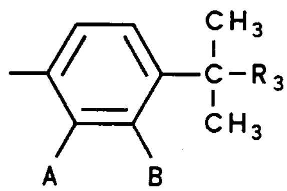

bedeutet, in der $R_3$ einen Rest der Formel $-CH_3$, $-CH_2OH$, -COOH oder der Formel -COOAlkyl bedeutet, in der die Alkyleinheit 1 bis 6 Kohlenstoffatome besitzt und unverzweigt oder verzweigt ist; A und B jeweils ein Wasserstoffatom oder eine Hydroxylgruppe bedeuten; mit den Maßgaben, daß mindestens einer der Reste A oder B ein Wasserstoffatom ist und einer der Reste A oder B kein Wasserstoffatom bedeutet, falls $R_3$ ein Rest der Formel $-CH_3$ ist; und pharmazeutisch verträgliche Salze und einzelne optische Isomere davon, b) eines oder mehrerer nichtionischer Netzmittel in einer Menge von 0.1 bis 6 Gewichtsprozent der Zusammensetzung, c) von Calciumcarbonat in einer Menge von 2 bis 50 Gewichtsprozent der Zusammensetzung.

**2.** Verfahren gemäß Anspruch 1, umfassend ferner das Pressen des Arzneimittels in eine feste Form der Dosierungseinheit.

**3.** Verfahren gemäß Anspruch 1 oder 2, wobei das Piperidinalkanol α-[4-(1,1-Dimethylethyl)phenyl]-4-(hydroxydiphenylmethyl)-1-piperidinbutanol ist.

**4.** Verfahren gemäß Anspruch 3, wobei das α-[4-(1,1-Dimethylethyl)phenyl]-4-(hydroxydiphenylmethyl)-1-piperidinbutanol in der Menge von etwa 60 mg verwendet wird.

**5.** Verfahren gemäß Anspruch 3, wobei das α-[4-(1,1-Dimethylethyl)phenyl]-4-(hydroxydiphenylmethyl)-1-piperidinbutanol in der Menge von etwa 120 mg verwendet wird.

**6.** Verfahren gemäß Anspruch 4 oder 5, wobei das nichtionische Netzmittel in einer Menge von 0.5 bis 4 Gewichtsprozent der Zusammensetzung verwendet wird.

**7.** Verfahren gemäß Anspruch 4 oder 5, wobei das nichtionische Netzmittel in einer Menge von 1 bis 3 Gewichtsprozent der Zusammensetzung verwendet wird.

**8.** Verfahren gemäß Anspruch 4, wobei das nichtionische Netzmittel Polysorbat 80 ist.

**9.** Verfahren gemäß Anspruch 5, wobei das nichtionische Netzmittel Polyethylenglykol ist.

**10.** Verfahren gemäß Anspruch 4 oder 5, wobei Calciumcarbonat in einer Menge von 2 bis 25 Gewichtsprozent der Zusammensetzung verwendet wird.

**11.** Verfahren gemäß Anspruch 4 oder 5, wobei das Calciumcarbonat in einer Menge von 12 bis 15 Gewichtsprozent der Zusammensetzung verwendet wird.

**12.** Verfahren gemäß Anspruch 4, umfassend die Vereinigung von Polysorbat 80 und Calciumcarbonat in Mengen von etwa 3 beziehungsweise 15 Gewichtsprozent der Zusammensetzung und umfassend ferner mikrokristalline Cellulose, vorgelatinierte Maisstärke, Natriumstärkeglykolat und Magnesiumstearat in Mengen von etwa 35, 30.5, 5 beziehungsweise 0.55 Gewichtsprozent der Zusammensetzung.

**13.** Verfahren gemäß Anspruch 5, umfassend die Vereinigung von Polyethylenglykol und Calciumcarbonat in Mengen von etwa 1 beziehungsweise 12 Gewichtsprozent der Zusammensetzung und umfassend ferner mikrokristalline Cellulose, HPMC, Magnesiumstearat und Siliciumdioxid in Mengen von etwa

60.4, 1.6, 1 beziehungsweise 0.3 Gewichtsprozent der Zusammensetzung.

**14.** Verfahren gemäß Anspruch 5, umfassend die Vereinigung von Polyethylenglykol und Calciumcarbonat in Mengen von etwa 1 beziehungsweise 12 Gewichtsprozent der Zusammensetzung und umfassend ferner mikrokristalline Cellulose, HPMC, Magnesiumstearat und Siliciumdioxid in Mengen von etwa 59, 3, 1 beziehungsweise 0.3 Gewichtsprozent der Zusammensetzung.

**15.** Verfahren zur Herstellung eines Arzneimittels in fester Form der Dosierungseinheit, umfassend die Vereinigung a) therapeutisch wirksamer Mengen von mindestens einer Piperidinalkanolverbindung, wie in Anspruch 1 gezeigt, b) von Calciumcarbonat in einer Menge von 2 bis 50 Gewichtsprozent der Zusammensetzung, und c) eines oder mehrerer kationischer Netzmittel in einer Menge von 0.1 bis 6 Gewichtsprozent der Zusammensetzung.

**16.** Verfahren gemäß Anspruch 15, umfassend ferner das Pressen des Arzneimittels in eine feste Form der Dosierungseinheit.

**17.** Verfahren gemäß Anspruch 15 oder 16, wobei die Piperidinalkanolverbindung α-[4-(1-1-Dimethylethyl)-phenyl]-4-(hydroxydiphenylmethyl)-1-piperidinbutanol ist.

**18.** Verfahren gemäß Anspruch 16 oder 17, wobei das kationische Netzmittel Cetylpyridiumchlorid ist.

**19.** Verfahren gemäß Anspruch 16 oder 17, wobei das kationische Netzmittel etwa 3 Gewichtsprozent der Zusammensetzung umfaßt.

**20.** Verfahren gemäß Anspruch 18, umfassend die Vereinigung von Cetylpyridiumchlorid und Calciumcarbonat in Mengen von etwa 3 beziehungsweise 15 Gewichtsprozent der Zusammensetzung und umfassend ferner mikrokristalline Cellulose, vorgelatinierte Maisstärke, Natriumstörkeglykolat und Magnesiumstearat in Mengen von etwa 35, 30.5, 5 beziehungsweise 0.55 Gewichtsprozent der Zusammensetzung.